# EUROPÄISCHE PATENTSCHRIFT

(11) **EP 0 793 942 B1**
(45) Veröffentlichungstag und Bekanntmachung des Hinweises auf die Patenterteilung: **01.02.2006**
(21) Anmeldenummer: 97250052.4
(22) Anmeldetag: 04.03.1997
(51) Int. Cl.: A61B 5/145, A61N 1/365

(54) **Vorrichtung zur Bestimmung der Blutsauerstoffsättigung**
Device for determining oxygen saturation of blood
Dispositif pour déterminer la saturation d'oxygène du sang

(30) Priorität: 04.03.1996 DE 19609410
(43) Veröffentlichungstag der Anmeldung: 10.09.1997
(73) Patentinhaber: BIOTRONIK Mess- und Therapiegeräte GmbH & Co Ingenieurbüro Berlin, 12359 Berlin (DE)
(72) Erfinder: Neumann, Andreas, Dipl.-Ing., 10969 Berlin (DE); Wieringa, Fokko, Ing., 6921 JA Duiven (NL); Hardeman, John, Ing., 6865 CP Doorwerth (NL)
(74) Vertreter: Lindner-Vogt, Karin L.

(56) Entgegenhaltungen:
- EP-A- 0 319 158
- EP-A- 0 443 495
- US-A- 4 653 498
- US-A- 4 807 630
- US-A- 4 880 304
- US-A- 5 193 543

## Beschreibung

Die Erfindung betrifft eine Vorrichtung der im Oberbegriff des Anspruchs 1 angegebenen Art.

Vorrichtungen zur Bestimmung der Blutsauerstoffsättigung, vielfach auch als (Puls-) Oximeter bezeichnet, sind in der Medizin seit längerem in Gebrauch.

Eine solche Vorrichtung, die speziell zur intrakardialen Verwendung ausgebildet ist und deren Meßwerte zur Frequenzsteuerung eines Herzschrittmachers herangezogen werden sollen, ist in DE 31 52 963 C1 beschrieben. Diese Vorrichtung umfaßt eine Meßsonde mit einer rot-emittierenden Leuchtdiode (LED) und einem Fototransistor sowie eine Ansteuer- und Signalwandlerschaltung, in der der empfangenen (Rot-) Lichtintensität proportionale Fotostrom unter Zuhilfenahme eines Referenzsignals in ein die Rot-Transmission des Blutes repräsentierendes elektrisches Signal umgewandelt wird. Dieses ist ein Ausdruck der Blutsauerstoffsättigung und damit des hämodynamischen Zustandes des Patienten und kann für die Schrittmachersteuerung genutzt werden.

Das Referenzsignal wird hier in einer zweiten Messung mit umgekehrter Spannungs-Polarität gewonnen, bei der durch eine hierzu vorgesehene Diode im Meßkreis der spezifische Meßsignalanteil eliminiert wird. Der Vergleich von Meß- und Referenzsignal liefert dann den spezifischen Signalanteil.

Eine von der Funktionsweise her ähnliche, aber zur nichtinvasiven Anwendung geschaffene, Vorrichtung ist in WO 92/21281 beschrieben. Hier ist ein zur Einführung eines Fingers ausgebildetes Gehäuse mit faseroptischer Verbindung zu einer entfernten Lichtquelle und einem entfernten Fotodetektor vorgesehen.

Ferner ist eine Vorrichtung mit den Merkmalen des ersten Teils des Anspruch 1 aus US-A- 4 807 630 bekannt.

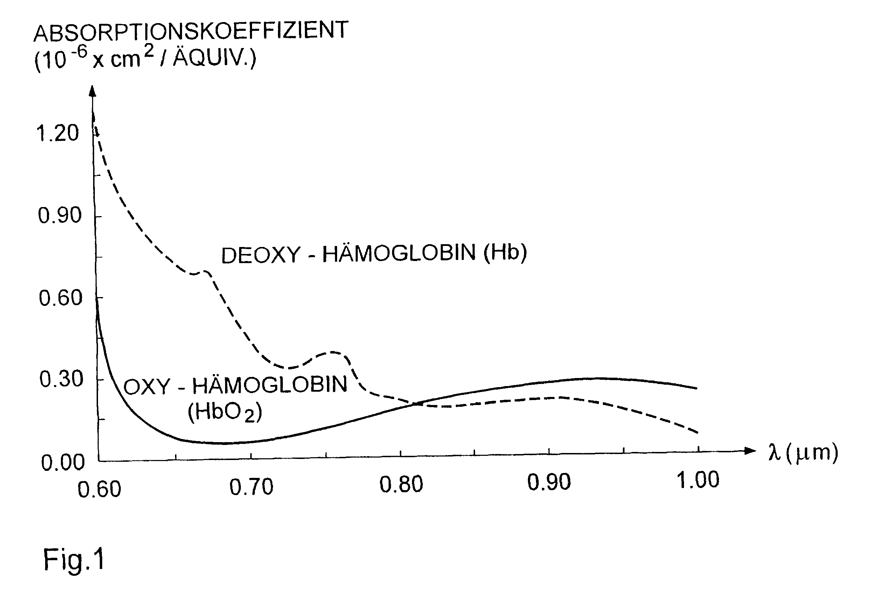

Die Nachteile des Stands der Technik werden nachfolgend anhand der Figuren 1 bis 3 dargestellt.

Der Erfindung liegt die Aufgabe zugrunde, eine Vorrichtung der eingangs genannten Gattung anzugeben, mit der eine weitgehend verfälschungsfreie Bestimmung der absoluten Blutsauerstoffsättigung mit geringem Meßfehler möglich und die variabel einsetzbar ist.

Diese Aufgabe wird durch eine Vorrichtung mit den Merkmalen des Anspruchs 1 gelöst.

Die Erfindung schließt den Gedanken ein, Mittel zur Bestimmung harmonischer Verzerrungen der empfangenen Strahlung und zum anderen die Bestimmung des Transmissionsgrades der empfangenen Strahlung vorzusehen. Dies ermöglicht es, einen Betrieb der Anordnung im nichtlinearen Bereich der Bauelemente (etwa bei Patienten mit sehr "duchscheinender" Haut), der zu einer Verfälschung der Meßwerte führen würde, zu erkennen und durch entsprechende Nachjustierung abzustellen. Hierzu ist eine Vergleichereinheit vorgesehen, in der das entsprechend verarbeitetete Empfangs-Pilotsignal mit dem Sende-Pilotsignal verglichen wird.

Die Verarbeitungsstufen können speziell jeweils ein Bandfilter und eine Gleichrichterstufe, deren Ausgangssignale - je nach Wahl des Filter-Durchlaßbereiches - ein Maß für die harmonischen Verzerrungen oder den Transmissionsgrad des durchstrahlten Gewebes sind.

Die Ergebnisse können dem Bediener angezeigt werden, woraufhin dieser eine manuelle Nachjustierung vornehmen kann.

Vorteilhafter ist aber das Vorsehen einer automatischen Justierung des Sendestromes nach Maßgabe der Vergleichsergebnisse auf einen Wert, bei dem die Detektoren in einem annähernd linearen Bereich arbeiten. Die ermittelten Werte bzw. die Justierstellung können patientenbezogen gespeichert und für spätere Messungen verwendet werden.

Im eigentlichen Meß-Signalweg (des unmodulierten Signalanteils) zur Bestimmung der Blutsauerstoffsättigung ist in einer einfachen Ausführung mit kostengünstigen Operationsverstärkern zunächst ein Tiefpaßfilter (etwa mit f_{go} = 40 Hz) mit einem erstem Operationsverstärker und ein Hochpaßfilter (etwa mit f_{gu} = 0,1 Hz - zur Eliminierung langsamer Nullpunktdrift) mit einem zweitem Operationsverstärker vorgesehen. Die Filter arbeiten vorzugsweise als Bessel-Filter mit minimaler Phasenverzerrung, und die Schaltung kann die Eigenschaften eines NIC-Konverters aufweisen.

Die Genauigkeit der Messung wird dadurch weiter erhöht, daß die aussendenden und/oder die empfangenden Bauelemente so ausgebildet und/oder derart mit Filtermitteln versehen sind, daß das Spektrum des empfangenen Lichts und dasjenige der empfangenen Infrarotstrahlung im wesentlichen keinen Überlappungsbereich aufweisen. Je ausgeprägter die Trennung der beiden Meßbereiche ist, desto größer kann angesichts der in Fig. 1 dargestellten Absorptionskurven die Meßgenauigkeit sein.

Das sichtbare Licht hat in Anbetracht dieser Kurven vorteilhafterweise ein Intensitätsmaximum bei einer Wellenlänge von etwa 660 nm und die Infrarotstrahlung ein Intensitätsmaximum bei einer Wellenlänge von etwa 950 nm.

Eine kostengünstige und kompakte und damit vielseitige Einsatzmöglichkeiten erschließende Realisierung der optoelektronischen Bauelemente besteht darin, daß die aussendenden Bauelemente mindestens je eine rot- und eine infrarotemittierende LED oder Laserdiode und die empfangenden Bauelemente ein rot- und ein infrarotempfindlicher Halbleiter-Fotodetektor sind. In einer speziellen, lichtstarken und den störenden Einfluß lokaler Inhomogenitäten des Gewebes bzw. des Blutstromes weitgehend eliminierenden Ausführung sind je drei rot- und infrarotemittierende LEDs bzw. Laserdioden vorgesehen.

Um die oben erwähnte Meßbereichstrennung sowie eine Ausschaltung störender kurzwelliger Strahlungsanteile bei leicht verfügbaren und kostengünstigen Standard-Bauelementen zu realisieren, kann dem das sichtbare Licht empfangenden Bauelement ein optisches Bandfilter und dem die Infrarotstrahlung empfangenden Bauelement ein Filter für kurzwellige Strahlungsanteile zugeordnet sein.

Die Vorrichtung kann im äußeren Aufbau für die Messung an der Körperoberfläche ausgebildet sein, indem die aussendenden und die empfangenden Bauelemente mit einem Abstandsbereich voneinander in einem (auch flexiblen) Gehäuse angeordnet sind, das zur Aufnahme eines Körperteils, insbesondere eines Ohrläppchens oder Fingers, eines Patienten im Abstandsbereich ausgebildet ist.

Es ist aber auch möglich, die aussendenden und die empfangenden Bauelemente mit einem Abstandsbereich voneinander in einer miniaturisierten Baugruppe anzuordnen, die zur intrakorporalen Messung, insbesondere im Herzen oder in einem größeren Gefäß, ausgebildet ist.

Die Vorrichtung eignet sich zur Steuerung eines in Abhängigkeit von der Blutsauerstoffsättigung zu steuernden Körperimplantates, insbesondere eines Herzschrittmachers.

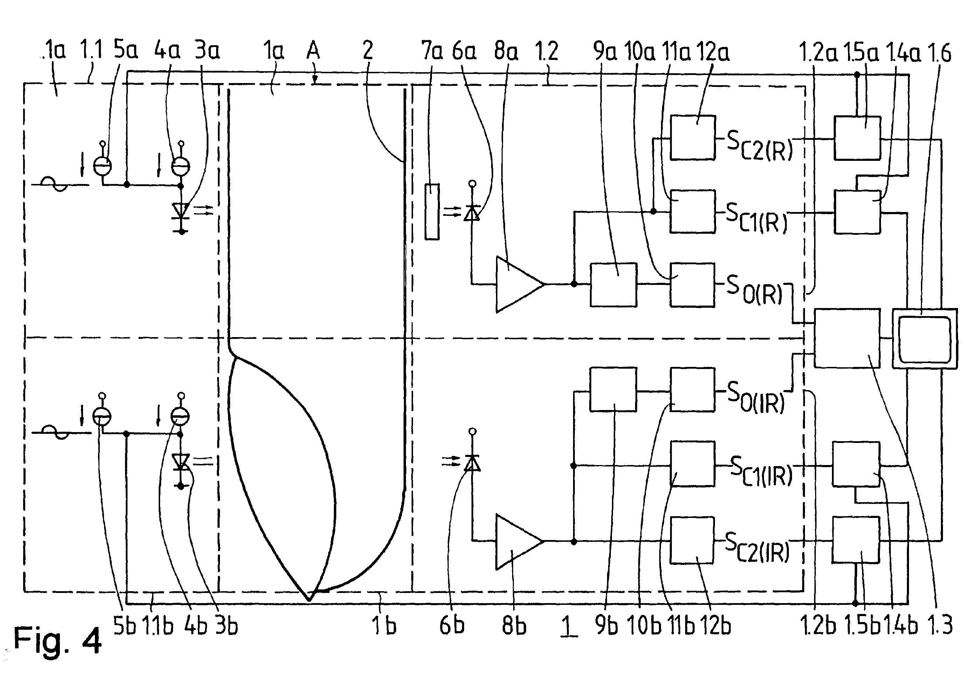

Nach der Wiedergabe der erwähnten allgemeinen Voraussetzungen nach dem Stand der Technik, zu dessen Verständnis die Figuren 1 bis 3 dienen, sind andere vorteilhafte Weiterbildungen der Erfindung in den Unteransprüchen gekennzeichnet bzw. werden anschließend zusammen mit der Beschreibung der bevorzugten Ausführung der Erfindung anhand der Figuren 4 bis 9 näher dargestellt. Es zeigen dabei im einzelnen:
Figur 1 eine grafische Darstellung des spektralen Absorptionsvermögens von Deoxy-Hämoglobin (Hb) und Oxy-Hämoglobin (HbO₂),
Figur 2 eine grafische Darstellung des Absorptionsvermögens von Blut bei 660 nm und bei 950 nm in Abhängigkeit von der Sauerstoffsättigung,
Figur 3 eine grafische Darstellung zum relativen Absorptionsvermögen von Blut in Abhängigkeit von der Sauerstoffsättigung,
Figur 4 ein vereinfachtes Blockschaltbild einer Vorrichtung zur Bestimmung der Blutsauerstoffsättigung nach einer Ausführungsform der Erfindung,
Figur 5 eine Detaildarstellung der Vorrichtung nach Fig. 4,
Figur 6a und 6b Darstellungen der Empfindlichkeitskurven der Licht- bzw. IR-Empfangs-Bauelemente mit vorgeschaltetem bzw. integriertem optischem Filter bei einer speziellen Ausbildung der in Fig. 4 gezeigten Vorrichtung,
Figur 7 eine Detaildarstellung der LED-Ansteuerung bei einer modifizierten Vorrichtung nach Fig. 4
Figur 8 eine Detaildarstellung der Auswertungsschaltung bei einer modifizierten Vorrichtung nach Fig. 4 und
Figur 9 eine schematische Darstellung zum Einsatz der er findungsgemäßen Vorrichtung bei der Steuerung eines Herzschrittmachers.

Zur Durchführung einer Absolutmessung der Blutsauerstoffsättigung können die im längerwelligen sichtbaren Bereich und im nahen Infrarot stark unterschiedlichen spektralen Absorptionskurven von Deoxy-Hämoglobin (Hb) und Oxy-Hämoglobin (HbO₂) dienen, die in Fig. 1 gezeigt sind.

In der Figur ist zu erkennen, daß das spektrale Absorptionsvermögen von Hb wie auch von HbO₂ bei 660 bis 670 nm nahezu konstant ist, wobei der Absorptionskoeffizient von Hb hier sehr viel höher ist als derjenige von HbO₂. Bei etwa 810 nm ist das Absorptionsvermögen gleich groß und oberhalb dieser Wellenlänge absorbiert HbO₂ stärker. Eine Vergleichsmessung bei einer Wellenlänge oberhalb von 810 nm gegenüber einer Primär-Messung bei etwa 660 nm ist daher für die Bestimmung des HbO₂-Anteils und damit der absoluten Blutsauerstoffsättigung mit hoher Genauigkeit vorteilhaft.

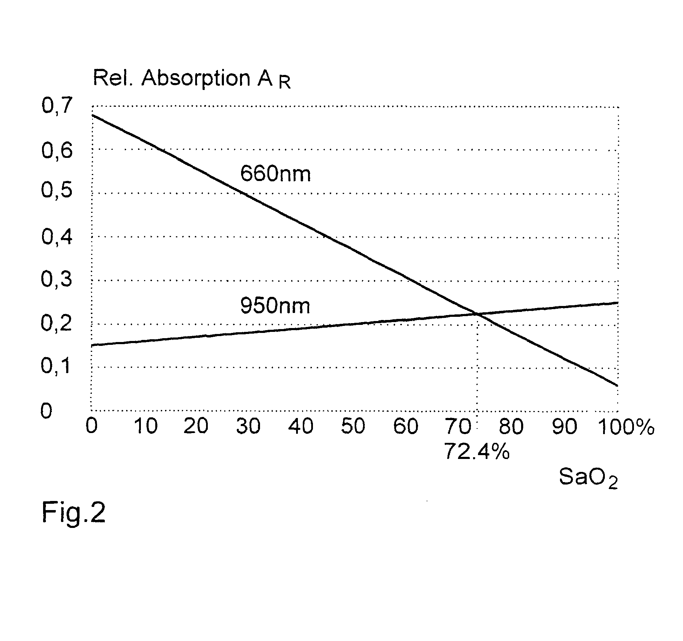

Fig. 2 gibt dabei eine grafische Darstellung des relativen Absorptionsvermögens bei 660 nm sowie bei 950 nm in Abhängigkeit von der Blutsauerstoffsättigung SaO₂.

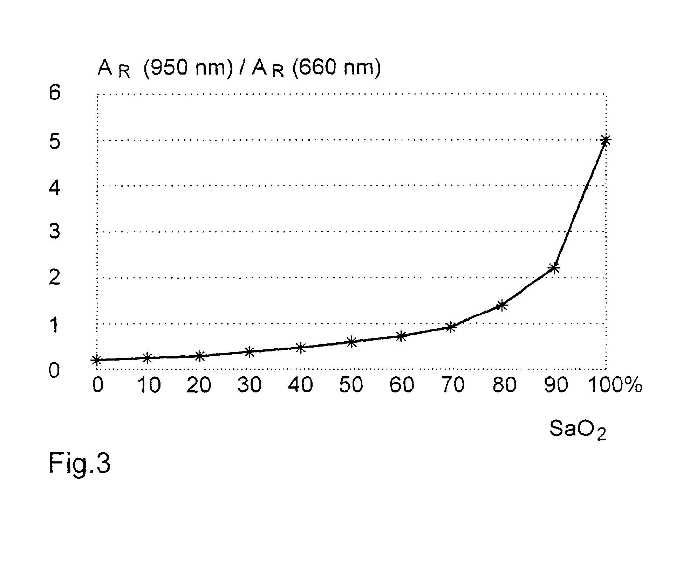

Fig. 3 gibt eine vom Informationsgehalt zu Fig. 2 äquivalente Darstellung, bei der der Quotient aus Infrarot(IR)-und Rot(R)-Absorption bei den genannten Wellenlängen über SaO₂ aufgetragen ist. Die Kreuze bezeichnen dabei Werte des Quotienten in 10%-Schritten, und diese wurden in der Figur durch Geraden verbunden; die eigentliche Funktionskurve ist eine Hyperbel.

Bei dem in Figur 4 wiedergegebenen vereinfachten Blockschaltbild einer Vorrichtung 1 als Ausführungsbeispiel der Erfindung wird der Vorgang einer extrakorporalen Bestimmung der Blutsauerstoffsättigung, in der Figur an einem Finger 2 verdeutlicht.

Die Vorrichtung umfaßt eine Sendestufe 1.1 mit einem senderseitigen Rot-Kanal 1.1a und einem senderseitigen Infrarot-Kanal 1.1b und eine Empfangsstufe 1.2 mit einem empfängerseitigen Rot-Kanal 1.2a und einem empfängerseitigen Infrarot-Kanal 1.2b. Die empfänger- und die senderseitigen Rot- bzw. Infrarot-Kanäle 1.1a und 1.2a bzw. 1.1b und 1.2b bilden zwei getrennte Meßkanäle 1a und 1b zur Ermittlung der Transmission bei sichtbarem Licht mit einer Wellenlänge von 660 bzw. bei Infrarotstrahlung mit einer Wellenlänge von 950 nm.

Die Sendestufe umfaßt eine bei 660 nm emittierende, d.h. rotleuchtende LED 3a mit einer Gleichspannungsversorgung 4a und einer Wechselspannungsversorgung 5a zur tonfrequenten (1 kHz-)Modulation des ausgesandten Lichtes im ersten Kanal 1a. Sie umfaßt analog eine bei 950 nm infrarot-emittierende LED 3b mit einer Gleichspannungsversorgung 4b und einer Wechselspannungsversorgung 5b zur 1 kHz-Modulation der ausgesandten IR-Strahlung im zweiten Kanal 1b.

Die Strahlung beider LEDs wird in einen Abstandsbereich A eines (in der Figur nicht gezeigten) Gehäuses, in den der Finger 2 eingeführt ist, emittiert und von einem jeweils der LED 3a bzw. 3b gegenüberliegenden Fotodetektor (einer PIN-Diode) 6a bzw. 6b aufgenommen. Dem Fotodetektor 6a im Rot-Kanal 1a ist ein optisches Bandfilter 7a vorgeschaltet. Die vom jeweiligen Fotodetektor 6a bzw. 6b gelieferten Signale durchlaufen nachfolgend analoge Verarbeitungswege. Sie werden zunächst in einem Verstärker 8a bzw. 8b verstärkt, wonach sich der Signalweg verzweigt.

Ein erster Signalweg führt jeweils über ein 40 Hz-Tiefpaßfilter 9a bzw. 9b und ein 0,1 Hz-Hochpaßfilter 10a bzw. 10b und liefert am Ausgang des letzteren ein Plethysmogramm- bzw. das eigentliche Meßsignal SO(R) bzw. SO(IR). Eine Quotientenbildung in einer nachgeordneten arithmetischen Verarbeitungsstufe 1.3 liefert daraus etwa einen Wert, aus dem anhand der in Fig. 3 gezeigten Kurve SaO₂ ablesbar ist.

Ein zweiter Signalweg führt jeweils über ein 1-kHz-Bandpaßfilter 11a bzw. 11b, an dessen Ausgang kontinuierlich je ein erstes Korrektursignal S_{C1 (R)} bzw. S_{C1 (IR)} zur Ermittlung der Transmissionseffizienz der Probe (des Fingers 2) bereitsteht. Dies geschieht durch einen Vergleich der empfangenen Amplitude des 1-kHz-Pilotsignals mit dem ursprünglichen, aufmodulierten Pilotsignal in jeweils einer ersten Pilotsignal-Verarbeitungs- und Vergleichereinheit 1.4a bzw. 1.4b.

Ein dritter Signalweg führt jeweils über ein 3-kHz-Bandpaßfilter 12a bzw. 12b, an dessen Ausgang je ein zweites Korrektursignal S_{C2 (R)} bzw. S_{C2 (IR)} bereitsteht, das zum Nachweis harmonischer Verzerrungen infolge nichtlinearen Betriebs der Bauelemente genutzt werden kann. Dies geschieht wiederum durch vergleichende Verarbeitung mit dem ursprünglichen 1-kHz-Signal in jeweils einer zweiten Pilotsignal-Verarbeitungs- und Vergleichereinheit 1.5a bzw. 1.5b.

Die Verarbeitungsergebnisse der Stufen 1.3. 1.4a. 1.4b. 1.5a und 1.5b werden einer Anzeigeeinheit 1.6 des Oximeters zugeführt, wo sie für den Bediener dargestellt werden, der daraufhin ggf. über eine entsprechende (nicht gezeigte) Bedieneinheit bestimmte Einstellungen, insbesondere den Ansteuerstrom der LEDs, verändern kann.

Die Auswertung der Transmissionseffizienz ermöglicht es auch, bei Messungen an einem Patienten zu verschiedenen Zeiten sowie für Messungen an einer Patienten-Population normierte Daten zur Verfügung zu haben. Für einen Patienten können dazu die in beiden Kanälen gewonnenen Transmissions-Daten in einer Patienten-Datei gespeichert und frühere Werte mit den Werten aktueller Messungen verrechnet werden.

Treten harmonische Verzerrungen auf, die mittels des 3-kHz-Filters nachgewiesen werden können - was insbesondere bei sehr hoher Umgebungshelligkeit oder sehr "durchscheinender" Haut des Patienten infolge Übersteuerung der Fotodetektoren 6a oder 6b vorkommen kann, so kann auch unmittelbar durch S_{C2 (R)} bzw. S_{C2 (IR)} eine (in der Figur nicht gezeigte) Steuereinrichtung betätigt werden, die die Gleichstromversorgung 4a bzw. 4b derart ansteuert, daß der LED-Strom für die LED 3a bzw. 3b in vorgegebenen Stufen kalibriert absenkt, bis keine Verzerrung mehr auftritt, d.h. kein Signal S_{C2 (R)} bzw. S_{C2 (IR)} mehr nachweisbar ist. Auch diese Einstellung kann für einen speziellen Patienten in dessen Patientendatei gespeichert werden, so daß bei einer späteren Untersuchung gleich mit dieser Einstellung begonnen werden kann.

Wird sowohl im zweiten als auch im dritten Signalweg kein Signal empfangen, so ist die Meßsonde nicht angeschlossen oder das Sondenkabel defekt, und der Bediener erhält auf dem Display 1.6 eine entsprechende Information.

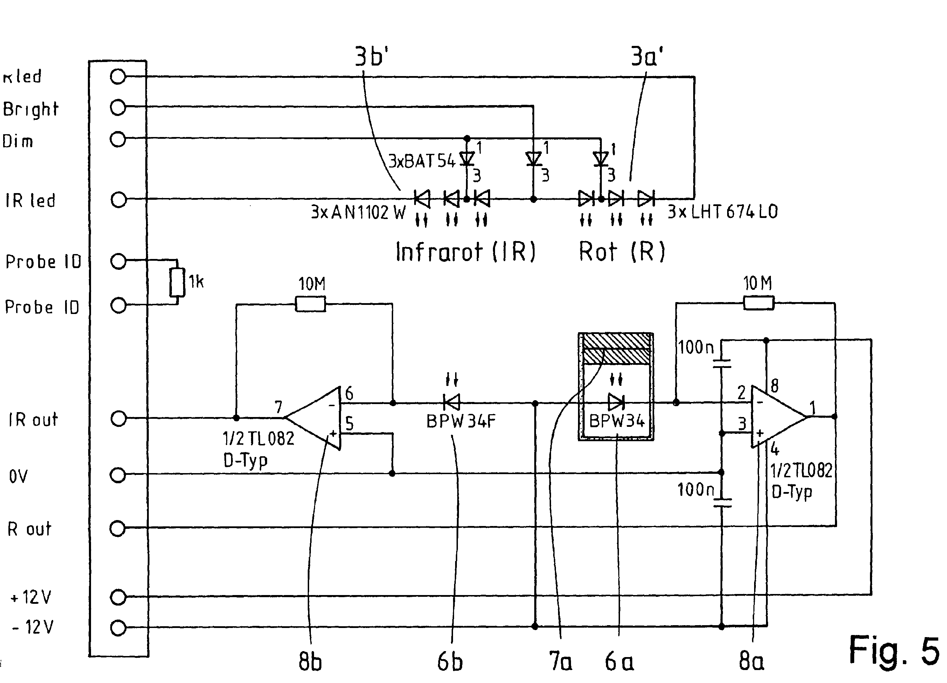

Ein genauere Darstellung der Schaltung und Bauelement-Bestückung der Sende- und der Empfangsstufe wird weiter unten unter Bezugnahme auf Fig. 5 bzw. die Figuren 7 und 8 gegeben.

In Fig. 5 ist ein Anschlußschema sowie Bauelement-Spezifikationen für die (durch Einsatz je dreier LED im Rot- und im Infrarot-Kanal leicht modifizierte) eigentliche Meßsonde gemäß Fig. 4 im Detail wiedergegeben. Die Bezugsziffern sind - bis auf die entsprechende Ersetzung der Ziffern 3a durch 3a' bzw. 3b durch 3b' - dieselben wie in Fig. 4, und die Schaltung wird als solche nicht nochmals beschrieben.

Die Anschlußbezeichnungen sind wie folgt zu verstehen:
Rled: Verbindung zur Rot-Stromquelle (4a, 5a in Fig. 4)
Bright: für den 3-LED-Betrieb +12V
Dim: für den 2-LED-Betrieb +12V, hat Priorität gegenüber Bright
IRled: Verbindung zur Infrarot-Stromquelle (4b, 5b in Fig. 4)
ProbeID: Reserve-Anschluß für eine spätere Kennzeichnung des Sondentyps
IRout: Ausgang des IR-Verstärkers 8b
OV: Masseanschluß für die Verstärker, separat mit zentraler Erde verbunden
Rout: Ausgang des Rot-Verstärkers 8a
+12V: positive Versorgungsleitung
-12V: negative Versorgungsleitung

Die vier erstgenannten Signale können auf einer Doppelleitung mit einfacher Abschirmung übertragen werden, deren Abschirmung mit einem zentralen 0V-Bezugspunkt verbunden ist. Minimales Übersprechen wird durch 180°-Phasenverschiebung zwischen dem Rot- und dem Infrarot-Pilotton erreicht.

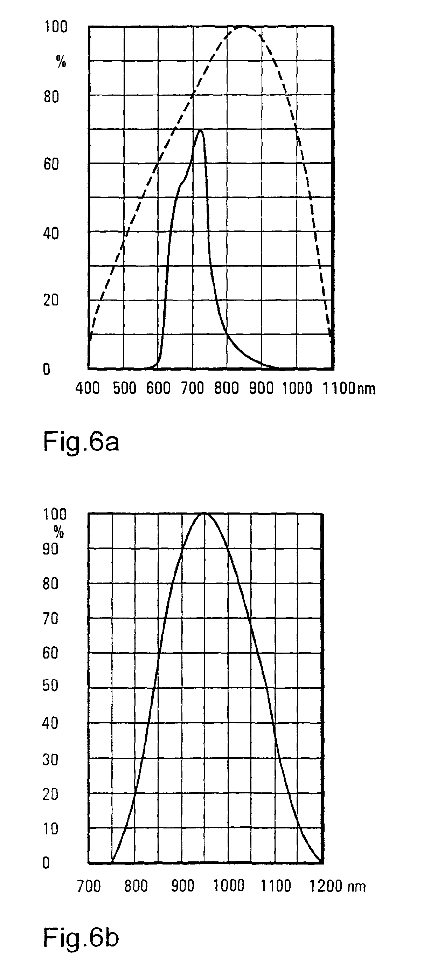

Die Figuren 6a und 6b geben Darstellungen der Empfindlichkeitskurven des Rot- bzw. des IR-Empfangs-Bauelementes 6a (mit vorgeschaltetem optischem Filter 7a) bzw. 6b (mit integriertem optischen Filter der in Fig. 4 gezeigten Vorrichtung.

Bei der in Fig. 5 spezifizierten Ausführung der Meßsonde wird als Detektor 6a im Rot-Kanal 1a eine PIN-Diode Siemens BPW34 verwendet, deren spektrale Empfindlichkeitskurve die Gestalt der gestrichelten Kurve in Fig. 6a hat. Es ist zu erkennen, daß der Detektor im Wellenlängenbereich von etwa 400 bis etwa 1100 nm empfindlich ist, während ein Nachweis nur um 660 nm benötigt wird und sowohl die Empfindlichkeit im kurz- als auch im langwelligeren Bereich die Meßgenauigkeit der Vorrichtung verschlechtert.

Der Detektor wird daher mit einem IR-Sperrfilter Edmund Scientific G39,424 sowie einem Filter zur Ausblendung kürzerer Wellenlängen versehen, wie es der Siemens BPO₂1 - Fotodetektor aufweist. Insgesamt ergibt sich mit dem Komposit-Filter 7a dann die durchgezogene Transmissionskurve.

Bei der in Fig. 6b gezeigten spektralen Empfindlichkeitskurve der als IR-Detektor verwendeten PIN-Diode Siemens BPW34F mit integriertem Tageslichtfilter ist zu erkennen, daß unterhalb 750 nm praktisch keine Transmsission auftritt, so daß eine ausgezeichnete Kanaltrennung Rot-Infrarot und nahezu völlige Unempfindlichkeit gegenüber Störungen durch sichtbares Licht erreicht wird.

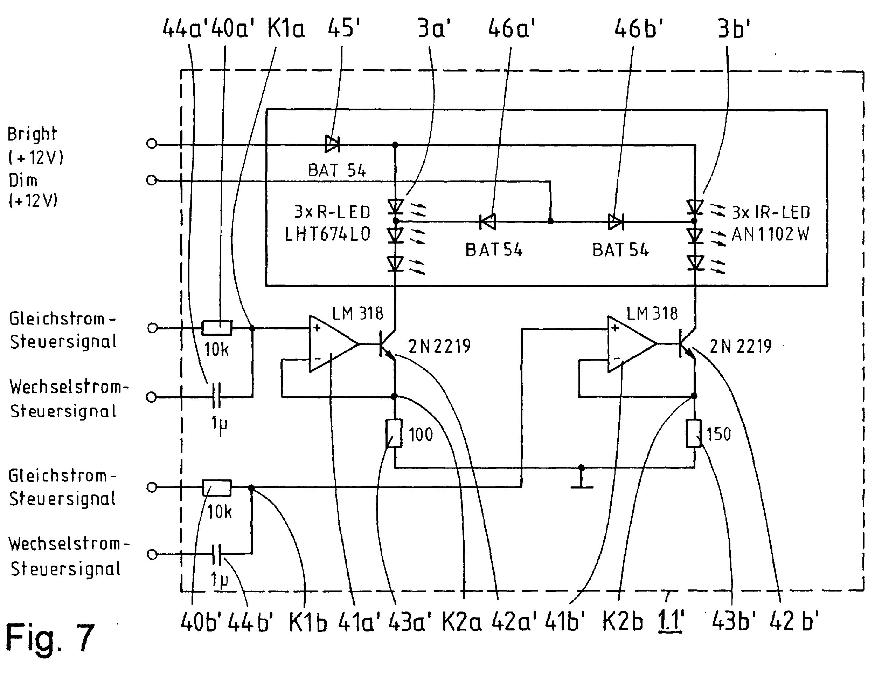

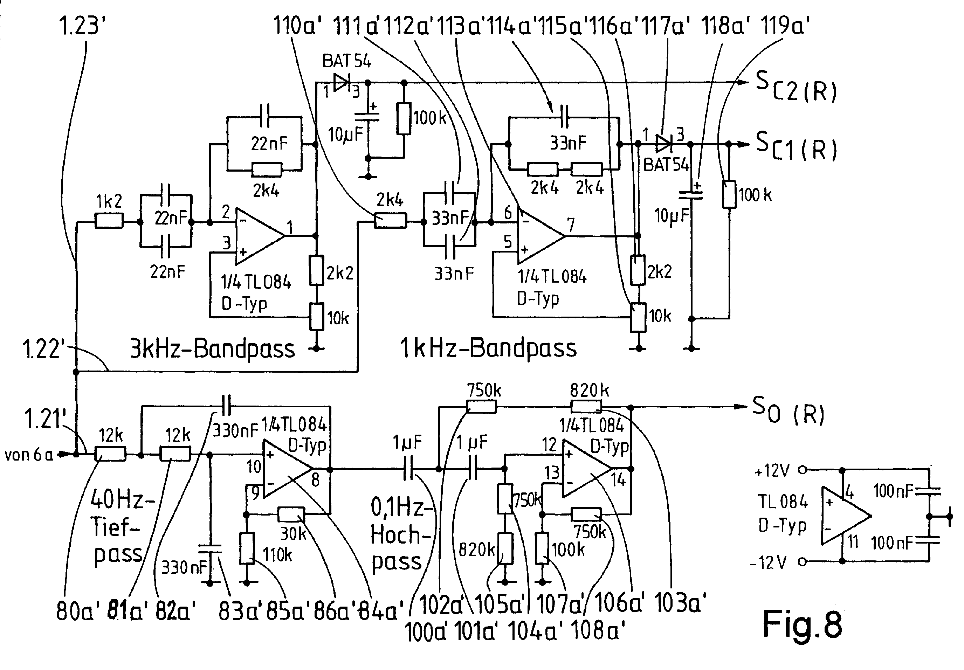

Bei der LED-Ansteuerung bei einem gegenüber der Vorrichtung nach Fig. 4 modifiziertem Pulsoximeter 1', von dem in der Detaildarstellung gemäß Fig. 7 nur die Sendestufe 1.1' gezeigt ist, kann die Empfangsstufe gemäß Fig. 4 bzw. der nachfolgend erläuterten Fig. 8 ausgebildet sein.

Die Sendestufe umfaßt als Strahlungsemitter - wie bereits in Fig. 5 gezeigt - je eine aus drei LEDs bestehende Rot-Sendergruppe 3a' und Infrarot-Sendergruppe 3b'. Die Spezifikation der Sende-Bauelemente ist (wie auch für die anderen Bauelemente) in der Figur angegeben.

Der Rot- und der Infrarot-Kanal verfügen über getrennte spannungsgesteuerte Stromquellen. Ein Gleichstromsteuersignal (10 mA/V für Rot, 15 mA/V für Infrarot) wird jeweils über einen Widerstand 40a' bzw. 40b' und einen Knoten K1a bzw. K1b dem nicht-invertierenden Eingang eines Operationsverstärkers 41a' bzw. 41b' zugeleitet, dessen invertierender Eingang jeweils über einen Knoten K2a bzw. K2b einerseits mit dem Emitter eines npn-Transistors 42a' bzw. 42b' und andererseits über einen Widerstand 43a' bzw. 43b' mit Masse verbunden ist. Ein Wechselstromsteuersignal (für das 1 kHz-Pilotsignal) wird über einen Kondensator 44a' bzw. 44b' ebenfalls über den Knoten K1a bzw. K1b dem nicht-invertierenden Eingang des Operationsverstärkers 41a' bzw. 41b' zugeführt.

Die Ausgänge der Operationsverstärker 41a' bzw. 41b' sind jeweils mit der Basis der Transistoren 42a' bzw. 42b' verbunden. Die Kollektoren der Transistoren 42a' und 42b' sind entweder über die (vollständige) Sendeelementgruppe 3a' bzw. 3b' und eine Diode 45' mit einer +12V-Versorgungsspannung ("Bright") oder - im Falle der Benutzung nur je zweier LEDs - über jeweils zwei LEDs der Sendeelementgruppe und je eine Diode 46a' bzw. 46b' mit der +12V-Versorgungsspannung ("Dim") verbunden.

Die Operationsverstärker 41a', 41b' steuern in Abhängigkeit von den angelegten Gleich- und Wechselstrom-Steuersignalen in an sich bekannter Weise über die Transistoren 42a', 42b' in Basisschaltung deren Kollektorstrom und damit den Betriebsstrom der LED-Gruppen 3a' und 3b'.

Bei einer Variante der Empfangsstufe, von der Fig. 8 eine Detaildarstellung speziell der Auswertungsschaltung, eines gegenüber der Vorrichtung nach Fig. 4 etwas abgewandelten Pulsoximeters gibt, sind beide Kanäle gleichartig aufgebaut. Es ist hier nur der Rot-Kanal 1a' dargestellt. Mit Fig. 4 übereinstimmend ist die Führung des von der PIN-Diode 6a kommenden Meßsignals über drei getrennte Signalwege, die in Fig. 8 mit 1.21', 1.22' und 1.23' bezeichnet sind und auf denen Ausgangssignale S_{O(R)}, S_{C1 (R)} bzw. S_{C2 (R)} geliefert werden.

Im Signalweg 1.21' ist eine aus einem Widerstand 80a', einem weiteren Widerstand 81a', einem zu diesem parallel geschalteten Kondensator 82a', einem den Widerstand 81a' von Masse trennenden Kondensator 83a', einem Operationsverstärker 84a' und zwei weiteren Widerständen 85a' und 86a' gebildete Tiefpaßfilter- und Verstärkerschaltung vorgesehen, wobei der nicht-invertierende Eingang des Operationsverstärkers 84a' über die zueinander in Reihe geschalteten Widerstände 80a' und 81a' mit dem Ausgang des Fotodetektors und über den Kondensator 83a' mit Masse, sein invertierender Eingang über den Widerstand 85a' mit Masse und sein Ausgang über den Widerstand 86a' mit Masse, über den Kondensator 82a' mit dem Verbindungspunkt zwischen den Reihen-Widerständen 80a' und 81a' sowie mit der nächsten Verarbeitungsstufe verbunden ist.

Die Darstellung in der rechten unteren Ecke von Fig. 8 zeigt die Verbindung des Operationsverstärkesr (sowie auch der weiteren, weiter unten beschriebenen Operationsverstärker) mit der Stromversorgung und - über zwei nicht mit Bezugsziffern versehene Kondensatoren - mit Masse.

Vom Ausgang des Operationsverstärkers 84a' gelangt das Signal in eine aus einem Kondensator 100a', einem weiteren Kondensator 101a', einer zu diesem parallel liegenden Reihenschaltung aus zwei Widerständen 102a', 103a', einer den Kondensator 101a' von Masse trennenden Serienschaltung aus zwei Widerständen 104a', 105a', einem Operationsverstärker 106a' und zwei weiteren Widerständen 107a' und 108a' gebildete Hochpaßfilter- und Verstärkerschaltung, bei der der nicht-invertierende Eingang des Operationsverstärkers 106a' über die zueinander in Reihe geschalteten Kondensatoren mit dem Eingang dieser Stufe und über die Widerstände 104a', 105a' mit Masse, sein invertierender Eingang über den Widerstand 107a' mit Masse und sein Ausgang über den Widerstand 108a' mit Masse und über die Serien-Widerstände 102a', 103a' mit dem Verbindungspunkt zwischen den Kondensatoren 100a', 101a' verbunden ist und gleichzeitig den Ausgang des ersten Signalweges 1.21' bildet.

Die gezeigte Operationsverstärkerschaltung realisiert - neben dem 40Hz-Tiefpaß und dem 0,1Hz-Hochpaß als Filter mit näherungsweiser Bessel-Charakteristik und niedrigen Pahsen-Verzerrungen - einen NIC-Konverter ("Transimpedance Amplifier"), mit dem die Nichtlinearität der optischen Bauelemente in gewisser Weise kompensiert wird.

Der zweite und dritte Signalweg 1.22' und 1.23' sind gleichartig aufgebaut und unterscheiden sich nur in der (in der Figur angegebenen) Dimensionierung der Baulemente, so daß in der Figur nur der zweite Signalweg mit Bezugsziffern versehen ist und hier nur dieser beschrieben wird.

Er enthält einen Widerstand 110a', eine zu diesem in Reihe geschaltete Parallelschaltung aus zwei Kondensatoren 111a', 112a' und einen Operationsverstärker 113a', dessen invertierender Eingang über die vorgenannten Elemente einerseits mit dem Eingang der Stufe und andererseits mit einer RC-Parallelschaltung 114a' verbunden ist und dessen nichtinvertierender Eingang über den Abgriff eines Potentiometers 115a' mit Masse verbunden ist.

Der Ausgang des Operationsverstärkers 113a' liegt einerseits über einen Widerstand 116a' und das Potentiometer 115a' an Masse und ist andererseits zum einen mit der RC-Parallelschaltung 114a' und zum anderen mit dem Eingang einer in Durchlaßrichtung geschalteten Diode 117a' verbunden. Deren Ausgang bildet - von Masse durch eine RC-Parallelschaltung aus einem Elektrolytkondensator 118a' und einem Widerstand 119a' getrennt - den Ausgang des Signalweges 1.22'.

Die Funktion des zweiten und dritten Signalweges wurde bereits oben unter Bezugnahme auf Fig. 4 erläutert. Mit der gezeigten Schaltung werden mit nur einem Operationsverstärker pro Stufe Filter hoher Güte realisiert.

Abweichungen von den gezeigten Schaltungen sind dem Fachmann jederzeit möglich, wobei auch das Prinzip des Nachweises harmonischer Verzerrungen über ein Pilotsignal modifizierbar ist.

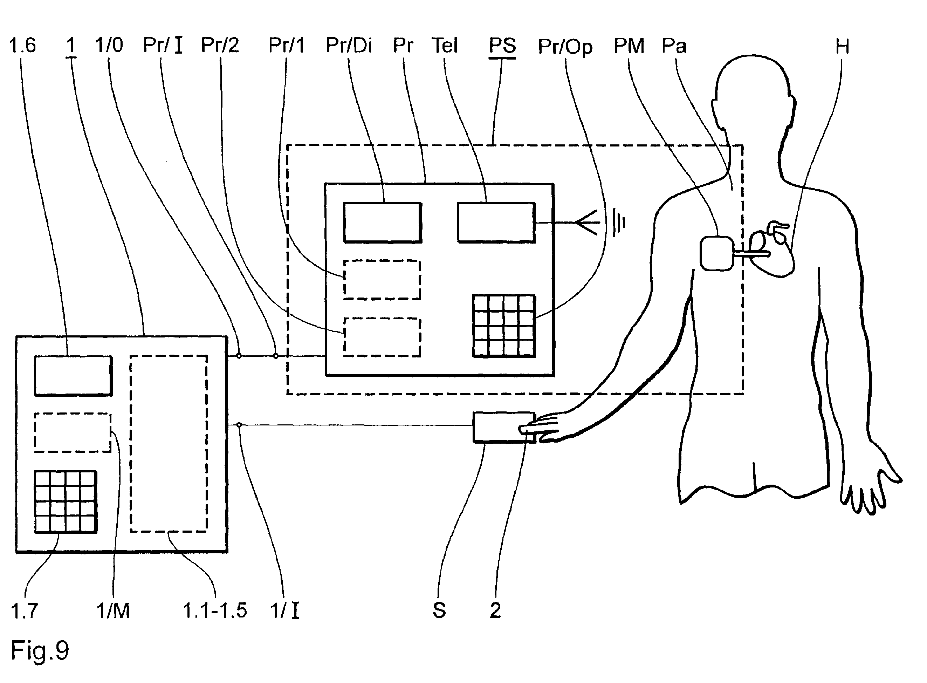

Figur 9 ist eine schematische Darstellung zum Einsatz des Pulsoximeters 1 für die Steuerung bzw. Programmierung eines Herzschrittmachersystems PS aus einem Programmiergerät Pr und einem in einen Patienten Pa implantierten Schrittmacher PM. Das Programmiergerät weist eine Bedieneinheit Pr/Op und eine Anzeigeeinheit Pr/Di auf, und innerhalb des Gerätes sind zwei Einstellfunktionen durch Blöcke Pr/1 bzw. Pr/2 symbolisiert. Schrittmacher PM und Programmiergerät Pr stehen über eine Telemetrieeinheit Tel, über die am Programmiergerät vorgenommene Betriebsparametereinstellungen an den Schrittmacher übertragen werden, miteinander in Verbindung.

Zum Pulsoximeter gehört eine Meßsonde S, die einen Finger 2 des Patienten Pa aufnimmt. Die Meßsonde ist an einen Eingang 1/I des Pulsoximeters angeschlossen, und dieses ist über einen Ausgang I/O mit einem Eingang Pr/I der Programmiereinheit Pr des Schrittmachersystems PS verbunden. Das Pulsoximeter weist eine (bereits in Fig. 4 gezeigte) Anzeigeeinheit 1.6 und eine Bedieneinheit 1.7 auf. Die in Fig. 4 einzeln darstellten Baugruppen 1.1 bis 1.5 sind in Fig. 9 zusammenfassend als ein (gestrichelt gezeichneter) Block symbolisiert, und weiterhin ist ein Meßwert- und Einstellungsspeicher 1/M vorgesehen.

Das Zusammenwirken des Pulsoximeters 1 mit dem Schrittmachersystem PS bei einer Routineuntersuchung zur Überprüfung der Funktionstüchtigkeit und der Programmparameter des Schrittmachers ist (im Sinne eines Beispiels) wie folgt:

Im Laufe der Überprüfung der Einstellungen des Schrittmachersystems wird über das Bedienfeld Pr/Op die erste extern, d.h. über die Programmiereinheit Pr, zu realisierende Einstellfunktion Pr/1 aufgerufen, die einen Bezug zum hämodynamischen Zustand des Patienten Pa hat, etwa die Einstellung der Stimulationsrate. Mit der bis zur Untersuchung gültigen Einstellung sowie weiteren im System vorgesehenen Grundeinstellungen wird eine Meßreihe gebildet. In jedem Schritt der Meßreihe wird ein Einstellwert über die Telemetrieeinheit Tel an den Schrittmacher PM übertragen und von diesem über eine am Programmiergerät Pr vorgegebene Zeitspanne das Herz H des Patienten Pa mit diesem Wert stimuliert.

Nach Verstreichen einer vorbestimmten Zeitspanne (die für das Pulsoximeter etwa durch Übertragung eines Auslösesignals durch das Programmiergerät signalisiert wird) wird durch das Pulsoximeter 1 über die Meßsonde S eine Messung der Blutsauerstoffsättigung vorgenommen, und der für den eingesetllten Stimulationsratenwert erhaltene Blutsauerstoffsättigungswert wird in Zuordnung zum Ratenwert im Programmiergerät gespeichert, wobei die Nummer der Messung in der Meßreihe oder direkt der Raten- bzw. Frequenzwert als Adresse dienen kann.

Danach wird der nächste Wert der Stimulationsrate aufgerufen und das beschriebene Vorgehen für diesen wiederholt.

Gleichzeitig wird bei den Messungen im Speicher 1/M auch der während der Messung automatisch eingestellte Sendestrom für die (in Figur 4 nicht gezeigten) LEDs in der Meßsonde S unter dem Patientennamen abgelegt, so daß bei späteren analogen Untersuchungen bei demselben Patienten sogleich von den korrekten Einstellungen der Sensoranordnung ausgegangen werden kann.

Nach Abschluß der Meßreihe - bei gleichbleibender Belastung des Patienten - wird (auf weiter unten genauer beschriebene Weise) im Programmiergerät automatisch der höchste Wert der Blutsauerstoffsättigung ermittelt, im internen Speicher 1/M des Pulsoximeters 1 unter dem Patientennamen abgelegt und der zugehörige Ratenwert über das Programmiergerät Pr als neuer, endgültiger Einstellwert für die mit der Meßreihe untersuchte Belastungsstufe des Patienten an den Schrittmacher PM übermittelt.

Auf völlig analoge Weise können, wenn im Rahmen der Untersuchung eine Überprüfung bzw. Eichung der Belastungs-Raten-Kennlinie eines frequenzadaptiven Schrittmachers vorgenommen werden soll, weitere Meßreihen bei veränderter Belastung des Patienten durchgeführt und ausgewertet und der optimierte Ratenwert für jeden Belastungszustand an den Schrittmacher als neue permanente Einstellung übermittelt werden. Im Schrittmacher werden die einzelnen Ratenwerte zusammen mit einer Kennung für den Aktivitäts-bzw. Belastungszustand, dem sie zuzuordnen sind, abgespeichert und stehen dann für den Normalbetrieb abrufbar zur Verfügung.

Ebenfalls grundsätzlich analog kann in einer weiteren Meßreihe oder Serie von Meßreihen die zweite Einstellung Pr/2, etwa die AV-Verzögerung eines Zweikammerschrittmachers PM (der unten genauer beschrieben wird) bei einem oder mehreren vorgegebenen Ratenwert(en), optimiert werden.

Nach der erfolgten Neueinstellung erfolgt die Steuerung des Schrittmachers dann derart, daß über einen ihm zugeordneten, implantierten Aktivitäts- oder Belastungssensor ein Signal abgegeben wird, aus dem die oben erwähnte Kennung für den Belastungszustand gewonnen wird, und daß der zugehörige gespeicherte Raten- und AV-Delay-Wert eingestellt wird. (Auch dies wird weiter unten genauer erläutert.)

Die Erfindung beschränkt sich in ihrer Ausführung nicht auf das vorstehend angegebene bevorzugte Ausführungsbeispiel. Vielmehr ist eine Anzahl von Varianten denkbar, welche von der dargestellten Lösung auch bei grundsätzlich anders gearteten Ausführungen Gebrauch macht.

## Patentansprüche

1. Vorrichtung (1) zur Bestimmung der Blutsauerstoffsättigung, mit mindestens einem sichtbares Licht sowie Infrarotstrahlung aussendenden Bauelement (3a, 3b; 3a', 3b') sowie mindestens je einem das sichtbare Licht und die Infrarotstrahlung nach Durchgang durch einen durchbluteten Körperbereich (2) empfangenden Bauelement (6a, 6b) und einer Steuer- und Auswertungseinrichtung (1.1, 1.2; 1.1') zur Steuerung des Betriebs der Vorrichtung und zur Bestimmung der Blutsauerstoffsättigung aus den von dem empfangenden Bauelement (6a, 6b) abgegebenen Ausgangssignalen, wobei
die Steuer- und Auswertungseinrichtung (1.1, 1.2; 1.1') Mittel (5a, 5b) zum Erzeugen und Anlegen einer Wechselspannung an das oder die aussendende (n) Bauelement(e) (3a, 3b; 3a', 3b') zur Modulation der emittierten Strahlung sowie mindestens eine Verarbeitungsstufe (11a, 12a, 11b, 12b) zur Auswertung des modulierten Anteils der empfangenen Strahlung aufweist **dadurch gekennzeichnet, daß** die Verarbeitungsstufe bzw. die Verarbeitungsstufen jeweils eine Vergleichereinheit (1.4a, 1.4b, 1.5a, 1.5b) zum Vergleich eines im Ergebnis der Verarbeitung des empfangenen Signals erhaltenen Signals (S_{C1 (R)}, S_{C1 (IR)}, S_{C2 (R)}, S_{C2 (IR)}) mit einem Ansteuersignal für die aussendenden Bauelemente (3a, 3b) aufweisen.

2. Vorrichtung nach Anspruch 1, **dadurch gekennzeichnet, daß** eine Verarbeitungsstufe (12a, 1.5a, 12b, 1.5b) zur Bestimmung harmonischer Verzerrungen der empfangenen Strahlung vorgesehen ist.

3. Vorrichtung nach Anspruch 1 oder 2, **dadurch gekennzeichnet, daß** eine Verarbeitungsstufe (11a, 1.4a, 11b. 1.4b) zur Bestimmung des Transmissionsgrades der empfangenen Strahlung vorgesehen ist.

4. Vorrichtung nach einem der vorangehenden Ansprüche, **dadurch gekennzeichnet, daß** die Verarbeitungsstufen jeweils ein Bandfilter und ein Gleichrichterelement aufweisen.

5. Vorrichtung nach einem der vorangehenden Ansprüche, **dadurch gekennzeichnet, daß** im Signalweg der Ausgangssignale (S_{O(R)}, S_{O(IR)} ) zur Bestimmung der Blutsauerstoffsättigung ein Tiefpaßfilter mit erstem Operationsverstärker (80a' bis 86a') und ein Hochpaßfilter mit zweitem Operatinsverstärker (100a' bis 108a') vorgesehen sind.

6. Vorrichtung nach Anspruch 5, **dadurch gekennzeichnet, daß** das Tief- und Hochpaßfilter Bessel-Charakteristik und die Operationsverstärkerschaltung NIC-Konverter-Charakteristik aufweisen.

7. Vorrichtung nach einem der Ansprüche 2 bis 6, **dadurch gekennzeichnet, daß** eine Anzeigeeinheit (1.6) für den Transmissionsgrad und/oder harmonische Verzerrungen und eine Vorrichtung zur, vorzugsweise selbsttätigen Justierung des Betriebsstromes der aussendenden Bauelemente (3a, 3a', 3b, 3b') vorgesehen ist.

8. Vorrichtung nach Anspruch 7, **dadurch gekennzeichnet, daß** eine Speichereinheit (1/M) zur patientenbezogenen Speicherung des ermittelten Transmissionsgrades und/oder der Justierstellung des Betriebsstromes vorgesehen ist.

9. Vorrichtung nach einem der vorangehenden Ansprüche, **dadurch gekennzeichnet, daß** je ein das sichtbare Licht und die Infrarotstrahlung empfangendes Bauelement (6a, 6b) vorgesehen ist und daß das das sichtbare Licht aussendende und das das sichtbare Licht empfangende Bauelement (3a, 6a; 3a', 6a') einen ersten Meßkanal (1a) sowie das die Infrarotstrahlung aussendende und das die Infrarotstrahlung empfangende Bauelement (3b, 6b; 3b', 6b') einen zweiten, vom ersten getrennten, Meßkanal (1b) bilden und die Steuer- und Auswertungseinrichtung (1.1, 1.2; 1.1') so ausgebildet ist, daß der erste und der zweite Meßkanal (1a, 1b) im wesentlichen gleichzeitig betrieben und die in beiden Meßkanälen abgegebenen Ausgangssignale im wesentlichen gleichzeitig verarbeitet werden.

10. Vorrichtung nach Anspruch 9, **dadurch gekennzeichnet, daß** je drei rot- und infrarotemittierende LEDs bzw. Laserdioden vorgesehen sind.

11. Vorrichtung nach einem der vorangehenden Ansprüche, **dadurch gekennzeichnet, daß** sie zur Steuerung eines in Abhängigkeit von der Blutsauerstoffsättigung zu steuernden Körperimplantates, insbesondere eines Herzschrittmachers (PM), ausgebildet ist.

## Claims

1. A device (1) for determining the blood oxygen saturation, having at least one component (3a, 3b; 3a', 3b') emitting visible light and infrared radiation, and at least one component (6a, 6b) receiving the visible light and the infrared radiation after passage through a blood-perfused body area (2), and a control and analysis unit (1.1, 1.2; 1.1') for controlling the operation of the device and for determining the blood oxygen saturation from the output signals output by the receiving component (6a, 6b), the control and analysis unit (1.1, 1.2, 1.1') having means (5a, 5b) for generating and applying an AC voltage to the emitting component(s) (3a, 3b; 3a', 3b') for modulating the emitted radiation and at least one processing stage (11a, 12a, 11b, 12b) for analyzing the modulated component of the received radiation,
**characterized in that** the processing stage(s) each have a comparator unit (1.4a, 1.4b, 1.5a, 1.5b) for comparing a signal (S_{C1 (R)}, S_{C1 (IR)}, S_{C2 (R)}, S_{C2 (IR)}), which is obtained as a result of the processing of the received signal, to a control signal for the emitting component (3a, 3b).

2. The device according to Claim 1,
**characterized in that** a processing stage (12a, 1.5a, 12b, 1.5b) is provided for determining harmonic distortions of the received radiation.

3. The device according to Claim 1 or 2,
**characterized in that** a processing stage (11a, 1.4a, 11b, 1.4b) is provided for determining the degree of transmission of the received radiation.

4. The device according to one of the preceding claims,
**characterized in that** the processing stages each have a band filter and a rectifier element.

5. The device according to one of the preceding claims,
**characterized in that** a low-pass filter having first operational amplifiers (80a' through 86a') and a high-pass filter having second operational amplifiers (100a' through 108a') are provided in the signal path of the output signals (S_{O (R)}, S_{O (IR)}) for determining the blood oxygen saturation.

6. The device according to Claim 5,
**characterized in that** the low-pass filter and high-pass filter have Bessel characteristic and the operational amplifier circuit has NIC converter characteristic.

7. The device according to one of Claims 2 through 6,
**characterized in that** a display unit (1.6) for the degree of transmission and/or harmonic distortions and a device for preferably automatic adjustment of the operating current of the emitting elements (3a, 3b; 3a', 3b') are provided.

8. The device according to Claim 7,
**characterized in that** a memory unit (1/M) for patient-related storage of the established degree of transmission and/or the adjustment setting of the operating current is provided.

9. The device according to one of the preceding claims,
**characterized in that** a component (6a, 6b) which receives the visible light and a component (6a, 6b) which receives the infrared radiation are provided and the component (3a, 6a'; 3a', 6a') emitting the visible light and receiving the visible light forms a first measuring channel (1a) and the component (3b, 6b; 3b', 6b') emitting the infrared radiation and receiving the infrared radiation forms a second measuring channel (1b), which is separate from the first, and the control and analysis unit (1.1, 1.2; 1.1') is implemented so that the first and the second measuring channels (1a, 1b) are operated essentially simultaneously and the output signals output in both measuring channels are processed essentially simultaneously.

10. The device according to Claim 9,
**characterized in that** three red-emitting and infrared-emitting LEDs and/or laser diodes are provided.

11. The device according to one of the preceding claims,
**characterized in that** it is implemented for controlling a body implant, particularly a pacemaker (PM), to be controlled as a function of the blood oxygen saturation.

## Revendications

1. Dispositif (1) pour déterminer la saturation en oxygène du sang, comprenant au moins un composant (3a, 3b ; 3a', 3b') émettant de la lumière visible et du rayonnement infrarouge ainsi qu'au moins un composant (6a, 6b) recevant la lumière visible et le rayonnement infrarouge après passage à travers une zone de corps (2) inaugurée et un dispositif de commande et d'analyse (1.1, 1.2 ; 1.1') pour la commande de l'exploitation du dispositif et pour la détermination de la saturation en oxygène du sang à partir des signaux de sortie émis par le composant (6a, 6b) récepteur,
le dispositif de commande et d'analyse (1.1, 1.2 ; 1.1') présentant des moyens (5a, 5b) pour générer et appliquer une tension alternative sur le ou le (s) composant(s) (3a, 3b ; 3a', 3b') émetteur(s) pour la modulation du rayonnement émis et d'au moins un niveau de traitement (11a, 12a, 11b, 12b) pour l'analyse de la partie modulée du rayonnement reçu,
**caractérisé en ce que** le niveau ou les niveaux de traitement présentent chacun une unité comparatrice (1.4a, 1.4b, 1.5a, 1.5b) pour la comparaison d'un signal (S_{C1 (R)}, S_{C1 (IR)}, S_{C2 (R)}, S_{C2 (IR)} obtenu comme résultat du traitement du signal reçu avec un signal d'activation pour les composants (3a, 3b) émetteurs.

2. Dispositif selon la revendication 1, **caractérisé en ce qu'**un niveau de traitement (12a, 1.5a, 12b, 1.5b) est prévu pour la détermination de distorsions harmoniques du rayonnement reçu.

3. Dispositif selon la revendication 1 ou 2,
**caractérisé en ce qu'**un niveau de traitement (11a, 1.4a, 11b, 1.4b) est prévu pour la détermination du degré de transmission du rayonnement reçu.

4. Dispositif selon l'une quelconque des revendications précédentes, **caractérisé en ce que** les niveaux de traitement présentent chacun un filtre de bande et un élément redresseur.

5. Dispositif selon l'une quelconque des revendications précédentes, **caractérisé en ce qu'**un filtre passe-bas avec un premier amplificateur opérationnel (80a' jusqu'à 86a') et un filtre passe-haut avec un second amplificateur opérationnel (100a' jusqu'à 108a') sont prévus dans le chemin des signaux de sortie (S_{O (R)}, S_{O (IR)}) pour la détermination de la saturation en oxygène.

6. Dispositif selon la revendication 5, **caractérisé en ce que** le filtre passe-bas et le filtre passe-haut présentent une caractéristique de Bessel et le circuit amplificateur opérationnel une caractéristique de convertisseur NIC.

7. Dispositif selon l'une quelconque des revendications 2 à 6, **caractérisé en ce qu'**une unité d'affichage (1.6) est prévue pour le degré de transmission et/ou des distorsions harmoniques et un dispositif pour l'ajustage de préférence automatique, du courant de service des composants (3a, 3a', 3b, 3b') émetteurs.

8. Dispositif selon la revendication 7, **caractérisé en ce qu'**une unité de mémorisation (1/M) est prévue pour la mémorisation spécifique au patient du degré de transmission déterminé et/ou de la position de réglage du courant de service.

9. Dispositif selon l'une quelconque des revendications précédentes, **caractérisé en ce qu'**un composant (6a, 6b) recevant la lumière visible et le rayonnement infrarouge est prévu à chaque fois et **en ce que** le composant émettant la lumière visible et le composant recevant la lumière visible (3a, 6a, 3a', 6a') forment un premier canal de mesure (la) et le composant émettant le rayonnement infrarouge et le composant recevant le rayonnement infrarouge (3b, 6b, 3b', 6b') forment un second canal de mesure (1b) séparé du premier, et l'unité de commande et d'analyse (1.1, 1.2 ; 1.1') est réalisée de telle sorte que le premier et le second canaux de mesure (1a, 1b) sont exploités sensiblement simultanément et les signaux de sortie émis dans les deux canaux de mesure sont traités sensiblement simultanément.

10. Dispositif selon la revendication 9, **caractérisé en ce qu'**à chaque fois trois LEDs et des diodes laser émettant en rouge et en infrarouge sont prévues.

11. Dispositif selon l'une quelconque des revendications précédentes, **caractérisé en ce qu'**il est réalisé pour la commande d'un implant de corps à commander en fonction de la saturation en oxygène du sang, en particulier d'un pacemaker (PM) .
